# EUROPEAN PATENT APPLICATION

(11) **EP 4 674 419 A1**
(43) Date of publication of application: **07.01.2026**
(21) Application number: 23940229.0
(22) Date of filing: 26.06.2023
(51) Int. Cl.: A61K 35/407, A61K 31/7105, A61P 1/16, C12N 5/0735, A61K 9/00

(54) **NEW USE OF EXOSOME SECRETED BY HEPATOCYTE LINEAGE CELLS DIFFERENTIATED VIA DIRECTED INDUCTION OF EMBRYONIC STEM CELLS**

(30) Priority: 05.06.2023 CN 202310658121
(71) Applicant: GUANGXIU-GAOXIN LIFE SCIENCES CO., LTD. HUNAN, Changsha, Hunan 410205 (CN)
(72) Inventor: LIN, Ge, Changsha, Hunan 410205 (CN); SUN, Yi, Changsha, Hunan 410205 (CN); LU, Guangxiu, Changsha, Hunan 410205 (CN); LI, Jie, Changsha, Hunan 410205 (CN)
(74) Representative: Hoffmann Eitle
(86) International application number: PCT/CN2023/102383
(87) International publication number: WO 2024/250348

(57) **Abstract**

The present invention relates to the new use of an exosome secreted by hepatocyte lineage cells differentiated via the directed induction of embryonic stem cells, and in particular relates to the use of the exosome in the preparation of a drug for treating liver diseases. The exosome is secreted by hepatocyte lineage cells differentiated via the directed induction of embryonic stem cells, and the hepatocyte lineage cells are hepatic progenitor cells and/or mature hepatocyte-like cells.

## Description

### RELATED APPLICATIONS

This application claims priority to Chinese patent application No. 2023106581214, filed on June 5, 2023, entitled "NEW USE OF EXOSOME SECRETED BY HEPATOCYTE LINEAGE CELLS DIFFERENTIATED VIA DIRECTED INDUCTION OF EMBRYONIC STEM CELLS", which is hereby incorporated by reference in its entirety.

### TECHNICAL FIELD

The present disclosure relates to the fields of biology and medicine, in particular to new use of exosome secreted by hepatocyte lineage cells differentiated via directed induction of embryonic stem cells.

### BACKGROUND

The liver is one of the important organs of the human body and plays an important role in metabolism. It performs various functions including glycogen storage, decomposition of red blood cells, synthesis of plasma proteins, and detoxification. Acute liver failure (ALF) is a syndrome caused by a variety of reasons, with sudden massive hepatocyte necrosis and/or severe liver dysfunction, which progresses to hepatic encephalopathy in a short period of time. It has an acute onset and high mortality rate (80%-97%), and can cause multiple organ dysfunction syndrome (MODS). It has remained a persistent challenge in the medical field. Liver transplantation is currently considered the most effective treatment, but it is expensive with few liver sources. Moreover, even if the transplant is successful, immunosuppressants must be taken for life, so it is necessary to develop new treatment strategies.

In view of this, the present disclosure is hereby proposed.

### SUMMARY

On this basis, according to various embodiments of the present disclosure, technical schemes of uses of exosomes are provided as follows.

In a first aspect of the present disclosure, the present disclosure provides use of exosomes in the manufacture of a medicament for treating a liver disease.

The exosomes are secreted by hepatocyte lineage cells differentiated via directed induction of embryonic stem cells. The hepatocyte lineage cells are hepatic progenitor cells and/or mature hepatocytes. The exosomes are rich in some nucleic acid components with therapeutic efficacy. The nucleic acid components include miR-302a-3p, miR-302b-3p, miR-302c-3p, miR-302d-3p, miR-302a-5p, miR-21-5p, etc.

In some embodiments, the liver disease is acute liver failure.

In some embodiments, the medicament includes the exosomes and pharmaceutically acceptable excipients.

In some embodiments, the medicament is in the form of a tablet.

In some embodiments, the medicament is in the form of a capsule.

In some embodiments, the medicament is in the form of granules.

In some embodiments, the medicament is in the form of a suspension.

In some embodiments, the medicament is in the form of an oral solution.

In some embodiments, the medicament is in the form of an injection.

In a second aspect of the present disclosure, an in vitro method for protecting hepatocytes is provided. The method including treating the hepatocytes to be protected with exosomes secreted by hepatic progenitor cells and/or mature hepatoid cells.

In some embodiments, the hepatocytes to be protected are hepatocyte cell lines such as L02, HepG2, HuH-7, or the like, or mouse primary hepatocytes (mPHs).

In some embodiments, the hepatocytes to be protected have cell damage induced by D-galactosamine (D-gal).

In some embodiments, the treatment is performed for 24 hours to 48 hours.

In some embodiments, the exosomes are used in a concentration of 10 µg/mL 100 µg/mL in the treatment.

Details of one or more embodiments of the present disclosure are set forth in the following description. Other features, objects, and advantages of the present disclosure will become apparent from the description and claims of the present disclosure.

### BRIEF DESCRIPTION OF THE DRAWINGS

The below will briefly describe drawings to be used in the description of embodiments of the present disclosure to more clearly illustrate the technical solutions in embodiments. Obviously, the drawings in the following description are only some embodiments of the present disclosure and other drawings can also be obtained by those skilled in the art based on these drawings without creative work.
FIG. 1 is a diagram showing the identification of nanoparticle tracking analysis (NTA) of hepatocyte exosomes induced according to an embodiment of the present disclosure.
FIG. 2 is an electron microscopic identification image of hepatocyte exosomes induced according to an embodiment of the present disclosure.
FIG. 3 is an identification diagram showing protein immunoblotting of hepatocyte exosomes induced according to an embodiment of the present disclosure.
FIG. 4 is a schematic diagram showing the effectiveness of hepatocyte exosomes induced according to an embodiment of the present disclosure to protect hepatocytes from damage in vitro.
FIG. 5 is a schematic diagram showing the effectiveness of hepatocyte exosomes induced according to an embodiment of the present disclosure in protecting mice with acute liver failure in vivo.
FIG. 6 shows a diagram of general comparison of liver tissues and their histopathology and a TUNEL staining diagram in an experiment of treating acute liver failure in mice with hepatocyte exosomes induced according to an embodiment of the present disclosure.
FIG. 7 shows immunoblotting results of liver tissue protein in an experiment of treating acute liver failure in mice using hepatocyte exosomes induced according to an embodiment of the present disclosure.
FIG. 8 shows qRT-PCR results of inflammatory genes in liver tissue in an experiment of treating acute liver failure in mice using hepatocyte exosomes induced according to an embodiment of the present disclosure.
FIG. 9 shows neutrophil staining results in liver tissue in an experiment of treating acute liver failure in mice using hepatocyte exosomes induced according to an embodiment of the present disclosure.
FIG. 10 shows miRNA sequencing results of hepatocyte exosomes induced according to an embodiment of the present disclosure, showing the enriched miRNAs therein in a pie chart.
FIG. 11 shows qPCR test results of intracellular miRNA content after co-incubation of hepatocyte exosomes induced according to an embodiment of the present disclosure with mPHs.
FIG. 12 is a schematic diagram showing the effectiveness of some high-abundance miRNAs in hepatocyte exosomes induced according to an embodiment of the present disclosure to protect hepatocytes in vitro.

### DETAILED DESCRIPTION

The technical solutions of the embodiments of the present disclosure will be described clearly and completely describes with reference to the accompanying drawings in the embodiments of the present application. The described embodiments are merely some rather than all of the embodiments of the disclosure. All other embodiments obtained by a person of ordinary skill in the art based on the embodiments of the present application without creative efforts shall fall within the protection scope of the present application.

Unless otherwise defined, all technical and scientific terms used herein have the same meaning as commonly understood by those skilled in the art to which the present application belongs. The terms used herein in the specification of the present application are for the purpose of describing specific embodiments only and are not intended to limit the present application.

### Terminology

Unless otherwise indicated or contradicted, terms or phrases used herein have the following meanings:
As used herein, the scope of the terms "and/or" and "or/and" as used herein includes any item in two or more associated listed items, and also includes any and all combinations of the associated listed items. The any and all combinations include a combination of any two associated listed items, any more associated listed items, or all associated listed items. It should be noted that when using at least two conjunction pairings selected from "and/or" and "or/and" to connect at least three items, the technical solution of the present application undoubtedly includes the solutions connected by "logical AND" and the solution connected by "logical OR". For example, the term "A and/or B" includes three parallel schemes: A, B, and "A + B". For another example, a technical solution of "A and/or B and/or C and/or D" includes any item in A, B, C, and D (i.e., a technical solution connected by "logical OR"), and also includes any and all combinations of A, B, C, and D, which means including a combination of any two items or any three items in A, B, C, and D, and also including a four-item combination of A, B, C, and D (i.e., a technical solution connected by "logical AND").

Unless otherwise specified, the terms "a plurality of", "multiple" "multiple times", "multi-" and the like related in the present disclosure mean greater than 2 or equal to 2 in number. For example, "one or more" means one or two or more.

As used herein, the terms "a combination thereof", "any combination thereof", and the like include all suitable combinations of any two or more of the listed items.

Herein, the word "suitable" in "suitable combination", "suitable mode", "any suitable mode", "any suitable combination", and the like, is defined by the ability to implement the technical solution of the present disclosure, solve the technical problem of the present disclosure, and achieve the expected technical effect of the present disclosure.

Herein, the terms "preferably", "better", "preferable", and "preferred" are only used to describe embodiments or examples with a better effect. It should be understood that these terms do not limit the scope of the present disclosure.

Herein, the terms "further", "still further", "specifically", and the like are used for descriptive purposes to indicate differences in contents, but should not be construed as limiting the scope of the present disclosure.

Herein, the terms "optionally" and "optional" refer to something that is not necessary, i.e., any one selected from the two parallel schemes in which the listed items is "present" or "absent". If multiple "optionally" appear in a technical solution, unless otherwise specified and in the absence of contradictions or interdependencies, each of the "optionally" is independent.

In the present disclosure, the terms "first", "second", "third", "fourth", etc. in "first aspect", "second aspect", "third aspect", "fourth aspect", etc. are used for descriptive purposes only and are not to be construed as indicating or implying a relative importance or quantity, nor as implying any indication of the importance or quantity of the technical features indicated. Moreover, the "first", "second", "third", "fourth", etc. are used merely for purposes of non-exhaustive enumeration and description, and should not be construed as a limitation of quantity in closed-ended manner.

In the present disclosure, the technical features described in open-ended manner include a closed-ended technical solution consisting of the listed features, and also include an open-ended technical solution including the listed features.

In the present disclosure, when referring to a numerical interval (i.e., a numerical range), unless otherwise specified, the distribution of values that are selectable within the numerical interval is considered to be continuous and includes both the endpoints of the numerical interval (i.e., the minimum and maximum values) and each value between these two endpoints. Unless otherwise specified, when the numerical interval only refers to the integers within the numerical interval, it includes the two endpoint integers of the numerical range, and each integer between the two endpoints. Herein, it is equivalent to directly listing each integer. For example, t is an integer selected from 1 to 10, indicating that t is any integer selected from the integer group consisting of 1, 2, 3, 4, 5, 6, 7, 8, 9 and 10. In addition, when multiple ranges are provided to describe features or characteristics, these ranges can be combined. In other words, unless otherwise specified, the ranges disclosed herein should be understood to include any and all sub-ranges included therein.

Unless otherwise specified, the temperature parameter in the present disclosure is allowed to be constant temperature treatment or to vary within a certain temperature interval. It should be understood that the constant temperature treatment allows the temperature to fluctuate within the accuracy range controlled by the instrument. It is allowed to fluctuate in a range such as ±5° C., 4° C., ±3° C., ±2° C., and ±1° C.

In the present disclosure, %(w/w) and wt% both represent weight percentage, %(v/v) refers to volume percentage, and %(w/v) refers to mass volume percentage.

All documents mentioned in the present disclosure are incorporated into the present application by reference, just as each document is cited separately as a reference. The entire content and purpose of the cited references in the present disclosure are incorporated by reference unless they conflict with the objectives and/or technical solutions of the present application. When a cited reference is related in the present disclosure, relevant technical features, terms, nouns, phrases, and the like in the cited reference are also incorporated herein. When a cited reference is related in the present disclosure, the examples, preferred embodiments and alternatives of the cited relevant technical features are also incorporated in the present application as references, but only to the extent that they enable the implementation of the present disclosure. It should be understood that when a reference conflicts with the description of the present application, the present application shall prevail or the reference shall be adaptively modified according to the description of the present application.

Stem cell therapy can reduce liver inflammation, improve scarring and renew liver cells, and is expected to become a new strategy for treating liver failure.

Embryonic stem cells and induced pluripotent stem cells are multipotent stem cells that have pluripotency and self-renewal ability and can proliferate indefinitely in vitro. Such pluripotent stem cells can differentiate into various cells of the body under certain conditions, thus becoming an excellent source of "seed cells" for regenerative medicine, and have broad development prospects in the fields of basic research in life sciences and regenerative medicine. Studies have confirmed that embryonic stem cells and induced pluripotent stem cells can be induced to differentiate into cells of the hepatocyte lineage, and have shown certain therapeutic effects in animal models of liver disease.

However, as a cell product, it still has many disadvantages in clinical application, such as the need for a continuous supply of a large number of cells with stable phenotypes, complicated and high-cost operations, and more importantly, safety issues brought about by the cells, such as immune rejection and tumorigenicity. This has given rise to exosomes, a new type of stem cell-based cell-free therapy.

The use of stem cell-derived exosomes to replace cell transplantation opens up a new perspective for the treatment of liver disease, and as a new type of cell-free therapy, it has unique advantages over cell therapy:

First, exosomes, as a natural nanocarrier, can deliver functional proteins, mRNA, miRNA and other substances to recipient cells, thereby regulating the functions of corresponding target cells, without the risks of abnormal differentiation, immune response and tumorigenicity that people worry about in cell transplantation.

Secondly, compared with cells, exosomes are small in size and have relatively simpler components, making them easier to produce and preserve.

Thirdly, different types of exosomes contain a variety of different bioactive components, which also have protective effects on the body in various diseases.

Therefore, based on the unique and inherent biological properties and flexible operability, exosomes are conducive to their clinical application, and also provide new ideas for the treatment of liver diseases.

Conventional exosomes used for the treatment of liver diseases can be found in, for example, CN 115040543A, which recites use of an exosome formulation in the preparation of a medicament for the treatment of liver failure, including: obtaining an ex vivo sample after intervening in an in vivo animal model of liver failure using the exosome formulation; and investigating the regenerative effect of the exosome formulation on liver tissue through the ex vivo sample, the exosome formulation contains exosomes derived from human hepatocytes or at least one miRNA derived from human hepatocytes as an active substance. Furthermore, the in vivo animal model of liver failure is a mouse acute liver failure model induced by carbon tetrachloride. The in vivo animal model of liver failure is two types of mouse acute liver failure models induced by acetaminophen. Whereas, we used exosomes secreted by liver progenitor cells or/and mature hepatoid cells induced and differentiated by embryonic stem cells to treat a mouse acute liver failure model induced by lipopolysaccharide (LPS)/D-gal. The results showed that the exosomes can inhibit the massive death of hepatocytes by blocking the apoptosis and programmed necrosis signaling pathways of hepatocytes, thereby alleviating local inflammatory reactions and the infiltration of inflammatory cells such as neutrophils, reducing liver tissue damage, and improving liver function. The miRNA sequencing showed that the exosomes were rich in nucleic acid components with therapeutic effects, including miR-302a-3p, miR-302b-3p, miR-302c-3p, miR-302d-3p, miR-302a-5p and miR-21-5p, which may be the main components that function in exosomes.

An embodiment of the present application provides use of exosomes in the manufacture of a medicament for treating a liver disease.

The exosomes are secreted by hepatocyte lineage cells differentiated via directed induction of embryonic stem cells, and the hepatocyte lineage cells are hepatic progenitor cells and/or mature hepatocytes.

The exosomes are rich in some nucleic acid components with therapeutic efficacy, including miR-302a-3p, miR-302b-3p, miR-302c-3p, miR-302d-3p, miR-302a-5p and miR-21-5p, etc.

In some embodiments, the liver disease is acute liver failure.

In some embodiments, the medicament includes the exosomes and pharmaceutically acceptable excipients.

In the present disclosure, "medicament" includes any agent, compound, composition or mixture that provides a physiological and/or pharmacological effect in vivo or in vitro, and often provides a beneficial effect. The range of physiological and/or pharmacological effects produced by the "medicament" in vivo is not particularly limited, and the medicament may be systemically effective or locally effective. The activity of the "medicament" is not particularly limited, and the medicament may be an active substance that can interact with other substances or an inert substance that does not interact with other substances.

In some embodiments, the medicament may be a liquid formulation or a solid formulation. Liquid formulation refers to a formulation containing a liquid phase, such as, by way of non-limiting examples, a solution, a suspension, an emulsion, or the like. Non-limiting examples of solid formulations are a tablet, a capsule, a granule, a pill, or the like.

In some embodiments, the medicament may be an oral formulation, an injection, a drop, a patch, a tube feed formulation, etc., depending on the mode of administration.

In some embodiments, the excipients selected may be different depending on the dosage form.

In the present disclosure, the excipients include, but are not limited to, mannitol, sorbitol, sodium metabisulfite, sodium bisulfite, sodium thiosulfate, cysteine hydrochloride, thioglycolic acid, methionine, vitamin C, EDTA disodium salt, sodium calcium EDTA, monovalent alkali metal carbonates, acetates, phosphates or aqueous solutions thereof, hydrochloric acid, acetic acid, sulfuric acid, phosphoric acid, amino acids, sodium chloride, potassium chloride, sodium lactate, xylitol, maltose, glucose, fructose, dextran, glycine, starch, sucrose, lactose, mannitol, silicon derivatives, cellulose and derivatives thereof, alginates, gelatin, polyvinylpyrrolidone, glycerol, Tween 80, agar, calcium carbonate, calcium bicarbonate, surfactants, polyethylene glycol, cyclodextrin, phospholipid materials, kaolin, tale, calcium stearate, and magnesium stearate.

In the present disclosure, the term "pharmaceutically acceptable" refers to any one or any suitable combination of those ligands, materials, compositions, and/or dosage forms that are suitable, within the scope of sound medical judgment, for administration to a patient and are commensurate with a reasonable benefit/risk ratio.

In the present disclosure, the term "pharmaceutically acceptable excipients" refer to pharmaceutically acceptable materials, compositions, or vehicles, such as liquid or solid fillers, diluents, excipients, solvents, or encapsulating materials. As used herein, the wording "pharmaceutically acceptable carrier" include buffers, sterile water for injection, solvents, dispersion media, coatings, antibacterial and antifungal agents, isotonic agents, absorption delaying agents, or the like, that are compatible with pharmaceutical administration. Each carrier must be "pharmaceutically acceptable" in the sense of being compatible with the other ingredients of the formulation and harmless to the patient. Suitable examples include, but are not limited to: (1) sugars, such as lactose, glucose, and sucrose; (2) starches, such as corn starch, potato starch, and substituted or unsubstituted β-cyclodextrin; (3) cellulose and derivatives thereof, such as sodium carboxymethylcellulose, ethyl cellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) phosphate buffered saline; and (21) other non-toxic compatible substances used in pharmaceutical formulations.

In some embodiments, the medicament includes an effective amount of the exosomes.

In the present disclosure, the term "effective amount" refers to the dose of the component corresponding to this term that achieves the treatment, prevention, alleviation and/or relief of a specific disease, disorder, and/or symptom in a subject. In the present disclosure, unless otherwise specified, this term refers to the dose that achieves the treatment, prevention, alleviation and/or relief of a liver disease, disorder, and/or symptom.

In the present disclosure, "subject" is an animal, preferably a mammal, and further preferably a human. A subject includes, but is not limited to, consumers of healthcare products, and patients with at least one of a disease, a disorder and a symptom. The subject of the present disclosure is preferably a mammal. The term "mammal" refers primarily to warm-blooded vertebrate mammals, including but not limited to, for example, cats, dogs, rabbits, bears, foxes, wolves, monkeys, deers, murine (e.g., rats and mice), pigs, cattle, sheep, horses, humans, etc, preferably primates, more preferably humans.

In some embodiments, the subject is a mammal.

In some embodiments, the subject is a human or a mouse.

In the present disclosure, the "patient" refers to an animal, preferably a mammal, more preferably a human. The term "mammal" refers primarily to warm-blooded vertebrate mammals, including but not limited to, for example, cats, dogs, rabbits, bears, foxes, wolves, monkeys, deers, murine, pigs, cattle, sheep, horses, humans, etc, preferably primates, more preferably humans.

### A second aspect of the present disclosure

The present disclosure provides an in vitro method for protecting hepatocytes is provided, the method including treating the hepatocytes to be protected with exosomes secreted by hepatic progenitor cells and/or mature hepatoid cells.

In some embodiments, the hepatocytes to be protected are hepatocyte cell lines such as L02, HepG2, or HuH-7, or mPH.

In some embodiments, the hepatocytes to be protected have cell damage induced by D-gal.

In some embodiments, the treatment is performed for 24 hours to 48 hours, for example, 24, 36, or 48 hours.

In some embodiments, the exosomes are used in a concentration of 10 µg/mL to 100 µg/mL, for example, 10, 20, 30, 40, 50, 60, 70, 80, 90, 100 µg/mL, in the treatment.

The embodiments of the present disclosure will be described in detail below with reference to the examples. It should be understood that these examples are only for illustrative purposes and not intended to limit the scope of the present disclosure. The experimental procedures without specific conditions noted in the following examples are preferably conducted according to the guidelines given in the present disclosure, and may be performed according to the experimental manual or routine conditions in the art, according to the conditions suggested by the manufacturer, or according to the experimental procedures known in the art.

In the examples described below, the measurement parameters related to the components of the raw materials may be slightly deviated from the weighing accuracy range, unless otherwise specified. When temperature and time parameters are related to, acceptable deviation caused by the instrument test accuracy or operational accuracy is allowed.

### I. Directed induction and differentiation of human embryonic stem cells into hepatocytes

### Hepatocyte induction is divided into five stages:

The first stage is the initial embryonic stem cell stage. Human embryonic stem cell (hESC) lines were resuscitated and seeded into culture flasks pre-coated with LN521 matrix. The medium was replaced with fresh NutriStem hESC XF medium daily. The cells were passaged every three days. After three passages, when the hESCs were in their optimal growth state, the medium was replaced with induction medium.

The second stage is the definitive endoderm cell stage. The embryonic stem cells were cultured in RPMI-1640 medium supplemented with 100 ng/ml human Activin A and 25 ng/ml Wnt3a. During the culture process, the medium was replaced with RPMI-1640 supplemented with 100 ng/ml human Activin A and 0.2-2% (v/v) fetal bovine serum (FBS).

The third stage is the hepatic progenitor cell stage. The cells were cultured in KO/DMEM medium supplemented with 50 ng/ml Keratinocyte Growth Factor and 2% (v/v) fetal bovine serum. During the culture process, the medium was replaced with KO/DMEM supplemented with 20% (v/v) Serum Replacement, 1 mM L-glutamine, 1% (v/v) non-essential amino acids, 0.1 mM β-mercaptoethanol, and 1% (v/v) dimethyl sulfoxide, directedly inducing differentiation of the cultured cells into hepatocyte lineage cells. The end of this stage yielded hepatic progenitor cells (HPCs).

The fourth stage is the hepatic progenitor cell expansion and culture stage. Cells were first cultured in basal medium DMEM/F12 supplemented with insulin-transferrin-selenium (ITS), nicotinamide, L-ascorbic acid 2-phosphate, human recombinant albumin, epidermal growth factor (EGF), glycogen synthase kinase 3 inhibitor (CHIR99021), transforming growth factor β receptor inhibitor (SB431542), lysophosphatidic acid (LPA), and sphingosine-1-phosphate (S1P) for 1 day. Subsequently, the cells were digested into single cells using TrypLE and passaged at a density of 1×10⁴ to 5×10⁴ cells/cm2. The culture was continued for 4 days, and the medium was replaced every 24 hours during subsequent culture. During this stage, the cells could also be digested and passaged for 1-5 generations using TrypLE. After expanding the cells to a certain number, the resulting hepatic progenitor cells were cryopreserved in CS10 for future use. When cells were urgently needed, the hepatic progenitor cells could be thawed, and only the maturation induction needed to be performed to obtain hepatocytes. This could significantly shorten the induction time. Alternatively, maturation induction could be performed directly without cryopreservation during this stage.

Insulin has a final concentration of 6 µg/mL, transferrin has a final concentration of 12 µg/mL, sodium selenite has a final concentration of 8 ng/mL, nicotinamide has a final concentration of 6 mM, L-ascorbic acid 2-phosphate has a final concentration of 35 µg/mL, human recombinant albumin has a final concentration of 45 µg/mL, epidermal growth factor (EGF) has a final concentration of 12 ng/mL, glycogen synthase kinase 3 inhibitor (CHIR99021) has a final concentration of 3 µM, transforming growth factor β receptor inhibitor (SB431542) has a final concentration of 2.5 µM, lysophosphatidic acid (LPA) has a final concentration of 2 µM, and sphingosine-1-phosphate (S1P) has a final concentration of 0.4 µM.

The fifth stage is the hepatocyte maturation stage. The expanded hepatic progenitor cells from the fourth stage were cultured in L-15 medium supplemented with 10% (v/v) FBS, 10 ng/ml hepatocyte growth factor, 20 ng/ml oncostatin, 0.05 µM Dexamethasone, and 2 mM L-glutamine to promote hepatocyte maturation, inducing them into mature hepatocyte like cells (HLCs). Two days before the end of hepatocyte-like cell induction, the fetal bovine serum in the medium was replaced with exosome-depleted serum for supernatant collection and exosome extraction, thus excluding interference from serum-derived exosomes.

### II. Extraction and Identification of Exosomes

### 1. Extraction of Exosomes:

The supernatant from cultured hepatocytes at the end of the third, fourth, and fifth stages was collected into clean centrifuge tubes and centrifuged at 300 g for 10 minutes at 4°C. After centrifugation, the supernatant was carefully pipetted and collected into clean centrifuge tubes, discarding the pellet. The supernatant was centrifuged at 2000 g for 20 minutes at 4°C. After centrifugation, the supernatant was carefully pipetted and collected into clean centrifuge tubes to remove residual cells and debris in the pellet. The supernatant was then centrifuged at 15,000 g for 30 minutes at 4°C. After centrifugation, the supernatant was carefully pipetted and collected into clean centrifuge tubes to remove larger vesicular substances such as apoptotic bodies and other possible large particulate matter. Finally, the supernatant collected from the previous step was ultracentrifuged at 100,000 g for 80 minutes at 4°C, and the supernatant was discarded. The pellet was resuspended in PBS and ultracentrifuged again at 100,000g × 80 minutes at 4°C for washing, and the supernatant was discarded. The collected pellet was resuspended in a small volume of PBS and stored at -80°C, or subjected directly to subsequent processing.

### 2. Identification of Exosomes:

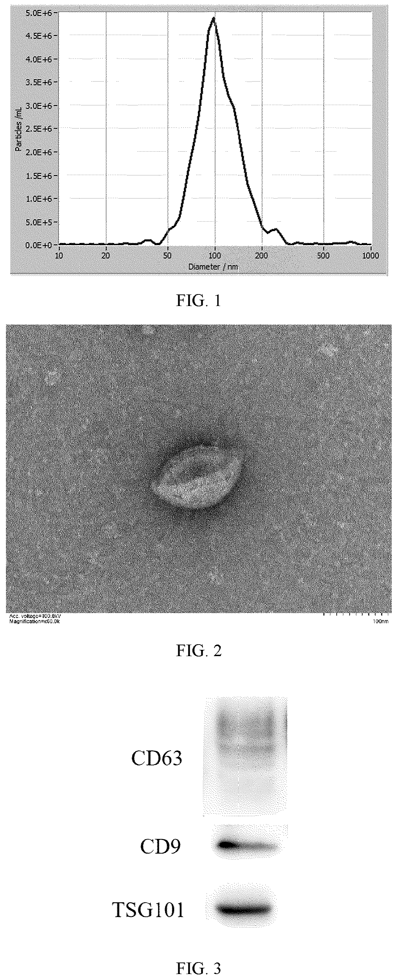

Taking the exosomes extracted from the supernatant of the third-stage hepatic progenitor cells as an example, the morphology, structure, particle size, and distribution of the exosomes were observed first using electron microscopy and NTA. The protein concentration of the exosomes was determined using a BCA protein assay kit. The expression of exosomal marker proteins CD9, CD63, and TSG101 was determined using western blot analysis, with the results shown in FIGS. 1, 2, and 3.

From FIG. 1, it can be seen that the diameter of the exosomes was concentrated at 50 to 200 nm, with a peak around 100 nm.

From FIG. 2, it can be seen that the morphology of the exosomes was cup/bowl-shaped vesicle structures, with intact particle morphology, and the vesicle size matched the NTA test.

From FIG. 3, it can be seen that the extracted exosomes possessed their specific markers TSG101, CD9, and CD63.

This indicated that the exosomes extracted were morphologically intact, their particle size and distribution were mainly concentrated within the defined range for exosomes, and they expressed exosome-specific marker proteins. Exosomes belong to the class of small extracellular vesicles (EVs), and were represented as EVs in the figures, where EVs from the third and fourth stages were designated hepatic progenitor cell exosomes (EVs-HPC), and EVs from the fifth stage were designated mature hepatocyte-like cell exosomes (EVs-HLC).

### 3. In vitro functional verification of exosomes:

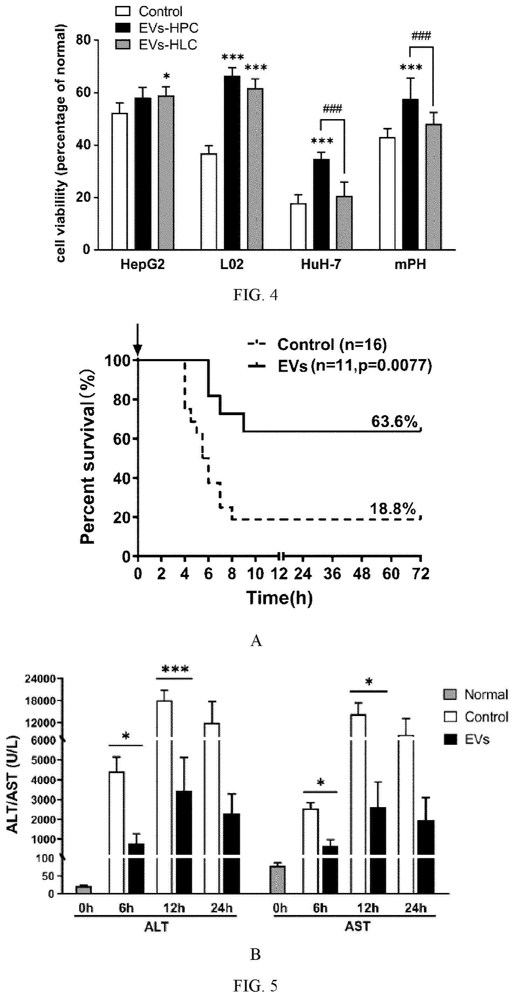

An in vitro cell model of D-galactosamine (D-gal)-induced injury was constructed using the human normal hepatocyte line L02, hepatocellular carcinoma cell lines HepG2 and HuH-7, as well as mPH in vitro. L02, HepG2, HuH-7, and mPH were seeded and cultured in RPMI-1640 medium containing 10% (v/v) fetal bovine serum (for L02), high-glucose DMEM medium (for HepG2 and HuH-7), and William's Medium E supplemented with 1 wt% L-glutamine (for mPH), respectively. When the confluence reached approximately 80%, the medium was replaced with the respective medium for each cell line containing 40 mM D-gal and 30 µg/mL exosomes. After 24 hours of treatment, the in vitro efficacy of the exosomes was detected using a lactate dehydrogenase cytotoxicity detection kit and is expressed using the following formula: Cell viability (%) = (Absorbance of treated sample - Absorbance of sample control well) / (Absorbance of enzyme activity of untreated cells - Absorbance of sample control well) × 100. The results are shown in FIG. 4.

According to FIG. 4, the cell viability of the exosome treatment group was significantly higher than that of the control group, indicating that exosomes derived from induced hepatocytes had a certain protective effect on D-gal-induced injured hepatocytes, and the effect of hepatic progenitor cell exosomes was slightly superior to that of mature hepatocyte-like cell exosomes.

Subsequently, the more effective hepatic progenitor cell exosomes (EVs-HPC) were selected for further validation in animal experiments.

### III. Acute Liver Failure Mouse Modeling and Treatment

1. Experimental Animals: SPF male Kunming mice weighing 18-22 g were purchased from Hunan Slac Jingda Experimental Animal Co., Ltd. (Production License No.: SYXK (Xiang) 2020-0006). The mice were fed a standard mouse diet with ad libitum access to filtered tap water. Ambient conditions were maintained at a temperature of 22°C ± 1°C and a humidity of 65% ± 5% under a 12-hour light/dark cycle. The animal experimental protocol was reviewed and approved by the Animal Ethics Committee of Central South University. Experimental procedures were strictly conducted in compliance with the National Standard of the People's Republic of China: Laboratory animal-Guideline for ethical review of animal welfare (GB/T 35892-2018).
2. Modeling and Treatment: Mouse acute liver failure models were constructed via intraperitoneal injection of LPS/D-gal. Subsequently, 200 µL of hepatic progenitor cell exosomes (EVs-HPC) or an equal volume of PBS buffer (designated as the EVs group and control group, respectively, in figures; the normal group is healthy mice) were administered via tail vein injection. Each mouse was injected at a dosage of exosomes secreted by 3.5 × 10⁶ hepatic progenitor cells. Survival was monitored for 72 hours for the Treatment group and control group, and the survival curve was plotted as shown in FIG. 5A. Results demonstrated that exosomes provided a protective effect, significantly improving survival rates and remarkably delaying death rate in hepatic failure mice.
3. Analysis of Exosome Treatment

### (1) Serum ALT/AST Levels and Histological Observation

Serum alanine aminotransferase (ALT) and aspartate aminotransferase (AST) levels were measured using a standard clinical autoanalyzer at Hunan Guangxiu Hospital. As shown in FIG. 5B, serum ALT and AST levels in the exosome treatment group were significantly lower than those in the control group, indicating markedly improved liver function.

### (2) Histopathological Analysis:

Fresh liver tissues were harvested and fixed in 4% paraformaldehyde for paraffin embedding. Embedded tissues were sectioned at 5 µm thickness and stained with hematoxylin and eosin (H&E). TUNEL staining was performed using the DeadEnd^{™} Colorimetric TUNEL System kit (Promega) to observe cell death in liver paraffin sections, and stained images were acquired under an optical microscope.

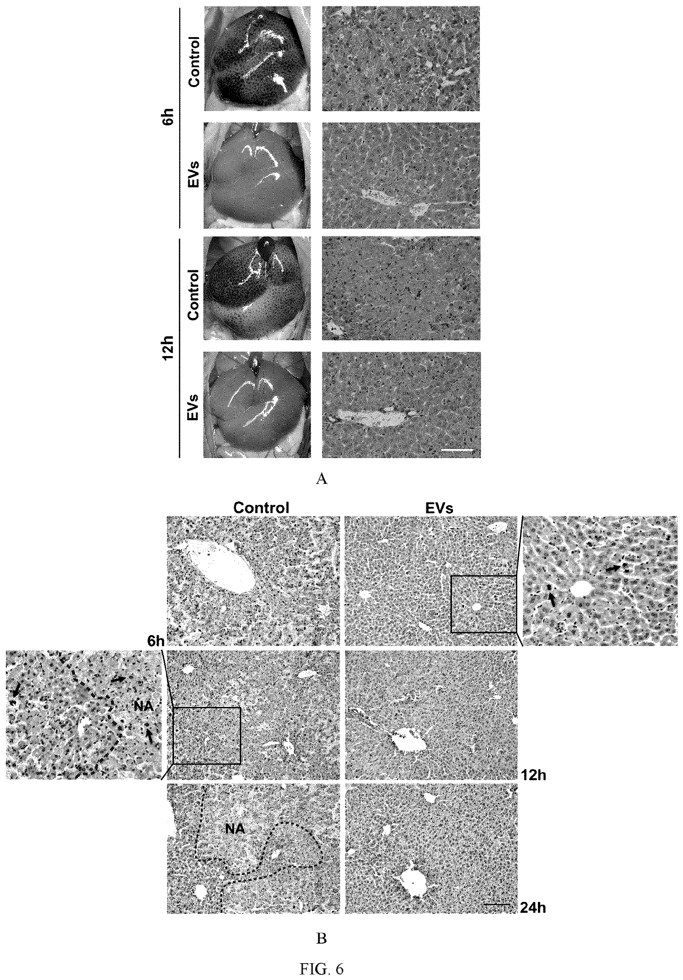

FIG. 6A presents H&E-stained liver tissue sections, while FIG. 6B shows TUNEL-stained sections. H&E staining of the liver tissue sections revealed diffuse hemorrhage, extensive necrotic areas, and massive inflammatory cell infiltration in the liver tissues in the control group, with severe disruption of hepatic lobe architecture. TUNEL staining showed large positively stained regions indicative of hepatocyte death (denoted as NA within dashed lines in figures) in the liver tissues in the control group. The exosome treatment group exhibited significantly attenuated liver injury, with only minor erythrocytes and scattered or focal hepatocyte death phenomena (indicated by arrows). Hepatic structure remained more intact, suggesting that hepatocyte exosomes has effect of inhibiting hepatocyte death and alleviate liver injury.

### (3) Western Blot Analysis of Apoptosis, Necroptosis, and Inflammatory Pathways

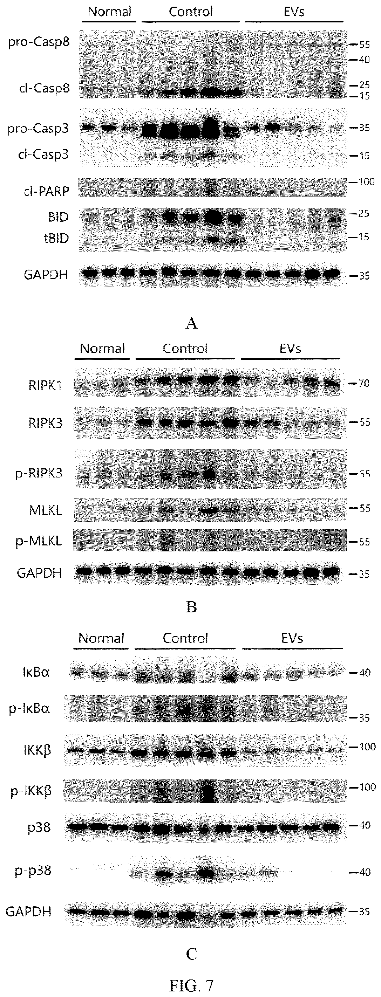

Mouse liver tissues was collected 6 hours post-modeling from control and exosome treatment groups, and protein was extracted for Western Blot to assess protein expression and activation in apoptosis-, necroptosis-, and inflammation-related signaling pathways. Results in FIG. 7 demonstrated that exosomes can reduce cleavage activation of apoptosis signaling pathway-related proteins Caspase-8/-3, PARP, and BID in liver tissues, having an inhibitory effect on apoptosis, as shown in FIG. 7); exosomes can inhibit phosphorylation activation of programmed necroptosis signaling pathway proteins RIPK3 and MLKL, counteracting programmed necrosis in hepatocytes, as shown in Fig. 7B, and exosomes further suppress activation of NF-κB and MAPK inflammatory related signaling pathways, with remarkably reduced phosphorylation of key molecules IKKβ, IκBα/β, and p38, as shown in FIG. 7C.

### (4) qRT-PCR Detection of Expression of Inflammatory Genes

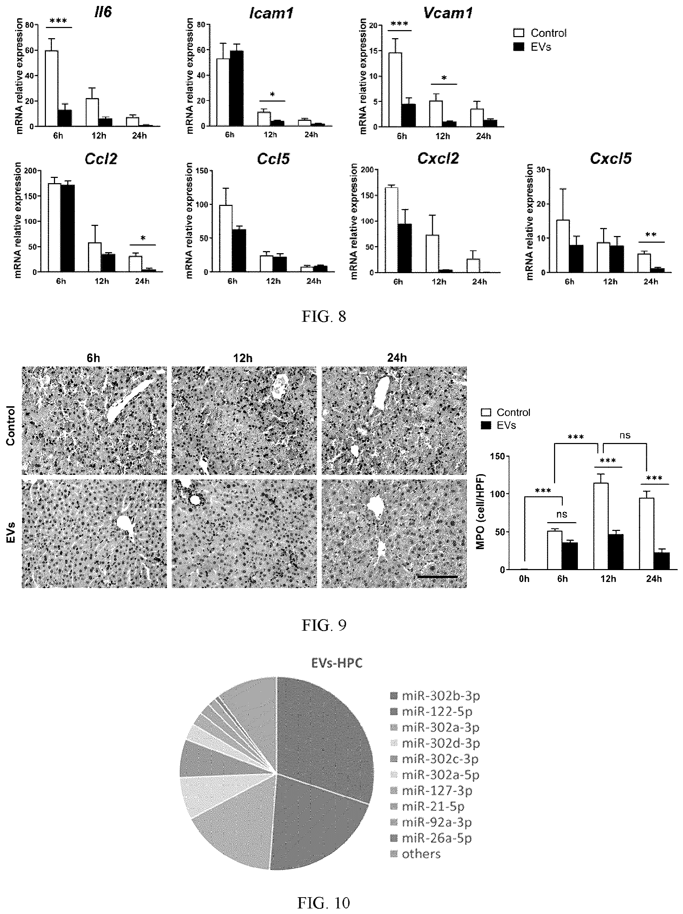

RNA was extracted from the liver tissues from control and exosome treatment groups using TRIzol. Reverse transcription was performed using 1 µg RNA per sample following the instructions of Reverse transcription kit. PCR reaction system contained 0.2 µL cDNA, 10 µL PCR Green Mix, 7.8 µL water, and 1 µL each of forward/reverse primers (the sequences of a pair of primers for validation were listed in Table 1). Cycling was performed as following: 95 °C for 5 min, followed by 45 cycles of 95 °C for 10 s, 58 °C for 10 s, and 72 °C for 10 s. We examined some inflammatory genes downstream of NF-κB and MAPK inflammation-related signaling pathways. As shown in FIG. 8, exosome treatment significantly reduced expression levels of multiple inflammatory genes compared to the control group.

**Table 1. Primer sequences for mouse inflammatory genes**

| Primer Name | Primer Sequence (5'-3') |
|---|---|
| mGapdh-F: SEQ ID NO.1 | AGGTCGGTGTGAACGGATTTG |
| mGapdh-R: SEQ ID NO.2 | TGTAGACCATGTAGTTGAGGTCA |
| mIl6-F: SEQ ID NO.3 | CCGGAGAGGAGACTTCACAG |
| mIl6-R: SEQ ID NO.4 | CAGAATTGCCATTGCACAAC |
| mCxcl2-F: SEQ ID NO.5 | CCAACCACCAGGCTACAGG |
| mCxcl2-R: SEQ ID NO.6 | GCGTCACACTCAAGCTCTG |
| mCxcl5-F: SEQ ID NO.7 | GTTCCATCTCGCCATTCATGC |
| mCxcl5-R: SEQ ID NO.8 | GCGGCTATGACTGAGGAAGG |
| mCcl2-F: SEQ ID NO.9 | TTAAAAACCTGGATCGGAACCAA |
| mCcl2-R: SEQ ID NO.10 | GCATTAGCTTCAGATTTACGGGT |
| mCcl5-F: SEQ ID NO.11 | AGATCTCTGCAGCTGCCCTCA |
| mCcl5-R: SEQ ID NO.12 | GGAGCACTTGCTGCTGGTGTAG |
| mIcam-F: SEQ ID NO.13 | GTGATGCTCAGGTATCCATCCA |
| mIcam-R: SEQ ID NO.14 | CACAGTTCTCAAAGCACAGCG |
| mVcam-F: SEQ ID NO.15 | GCTCTGGGAAGCTGGAACG |
| mVcam-R: SEQ ID NO.16 | GGAGCCAAACACTTGACCGT |

### (5) Analysis of Neutrophil Infiltration

Since acute inflammation is predominantly characterized by neutrophil infiltration. To reflect inflammatory cell infiltration within the tissues, myeloperoxidase (MPO) (an enzyme unique to neutrophils) staining was performed on liver tissues. As shown in FIG. 9, neutrophil infiltration was observed in liver tissues 6 hours after liver failure, peaking at 12 hours post-modeling. However, the number of neutrophils in the treatment group was remarkably reduced, with particularly significant differences at 12 and 24 hours post-injury, indicating that exosomes can reduce neutrophil infiltration.

### IV. Analysis of Active Components in Hepatocyte Exosomes

### 1. Small RNA (miRNA) Sequencing of Exosomes:

To identify possible functional components within exosomes, the extracted EVs-HPC and EVs-HLC were submitted to Shenzhen Bgi Genomics Co., Ltd. for miRNA sequencing. As shown in FIG. 10, the result demonstrated that both EVs-HPC and EVs-HLC were enriched with miRNAs including miR-302a-3p, miR-302b-3p, miR-302c-3p, miR-302d-3p, miR-302a-5p, and miR-21-5p. Notably, miR-302 family members accounted for nearly 60% of total miRNAs in EVs-HPC, with family member miR-302b-3p being the highest. miR-21-5p, a known anti-apoptotic miRNA, showed high abundance in both EVs-HPC and EVs-HLC. Additionally, miR-122-5p which ranked among the top 10 miRNAs was a hepatocyte-specific miRNA, confirming that the exosomes we extracted were derived from cells of the hepatocyte lineage cells.

### 2 Transferabilityof Exosomal miRNAs to Target Cells

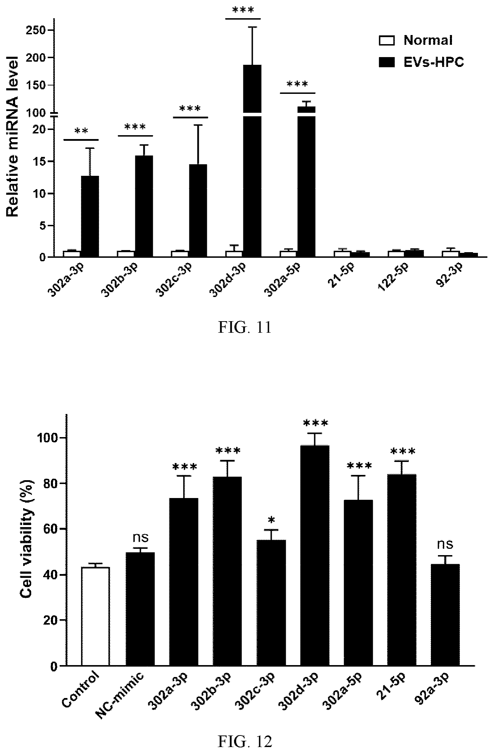

To verify that exosomal miRNAs could enter target cells via exosome uptake, EVs-HPC were co-cultured with mPHs. After 24 hours, miRNA was extracted from mPH and subjected to stem-loop reverse transcription and qPCR detection. The sequences of the primers for validation are shown in Table 2. The results are shown in FIG. 11, showing that mPHs, which expressed almost no miR-302 family members at baseline, includes significantly increased miR-302 family members after EV co-culture. Particularly, miR-302d-3p and miR-302a-5p increased by about over one hundred times, and miR-302b-3p increased by more than 15 times. In contrast, miR-21-5p, miR-122-5p, and miR-92-3p, which had high baseline expression in mPHs, showed no significant amount changes after co-cultured. These results suggested that exosomes delivered specific miRNAs therein via uptake by the target cells, potentially altering intracellular miRNA contents of the target cells and achieving the purpose of altering certain biological functions of the target cells.

**Table 2. sequences of miRNA primers**

| Primer Name | Primer Sequence (5'-3') |
|---|---|
| U6-F: SEQ ID NO.17 | CTCGCTTCGGCAGCACA |
| U6-R: SEQ ID NO.18 | AACGCTTCACGAATTTGCGT |
| hsa-miR-302a-3p_3sl: SEQ ID NO.19 | GTCCTCCTCTCCTCTCCTCTCATGAGGAG GACTCACCA |
| hsa-miR-302a-3p_3F: SEQ ID NO.20 | AGGCTAAGTGCTTCCATGTTT |
| hsa-miR-302a-3p_3R: SEQ ID NO.21 | TCCTCCTCTCCTCTCCTCTC |
| hsa-miR-302b-3p_3sl: SEQ ID NO.22 | GGCGTTGTGTTGTGTTGTGTTATCACAAC GCCCTACTA |
| hsa-miR-302b-3p_3F: SEQ ID NO.23 | AGGGTAAGTGCTTCCATGTTT |
| hsa-miR-302b-3p_3R: SEQ ID NO.24 | GCGTTGTGTTGTGTTGTGTT |
| hsa-miR-302c-3p_3sl: SEQ ID NO.25 | GGCGTTGTGTTGTGTTGTGTTATCACAAC GCCCCACTG |
| hsa-miR-302c-3p_3F | SEQ ID NO.23 |
| hsa-miR-302c-3p_3R | SEQ ID NO.24 |
| hsa-miR-302d-3p_2sl: SEQ ID NO.26 | GGAGAGGAGAGGAAGAGGGAAATCTCC TCTCCACACTC |
| hsa-miR-302d-3p_2F | SEQ ID NO.23 |
| hsa-miR-302d-3p_2R: SEQ ID NO.27 | GAGAGGAGAGGAAGAGGGAA |
| hsa-miR-302a-5p_3sl: SEQ ID NO.28 | GTGTCTGGGTGTGCTGTATTGATCCCAG ACACAGCAAG |
| hsa-miR-302a-5p_3F: SEQ ID NO.29 | AGGCACTTAAACGTGGATGTA |
| hsa-miR-302a-5p_3R: SEQ ID NO.30 | TGTCTGGGTGTGCTGTATTG |
| hsa-miR-122-5p_3sl: SEQ ID NO.31 | GGAGATGGGAGAGGGAGTAGGATCCCAT CTCCCAAACA |
| hsa-miR-122-5p_3F: SEQ ID NO.32 | GGGTGGAGTGTGACAATGG |
| hsa-miR-122-5p_3R: SEQ ID NO.33 | GAGATGGGAGAGGGAGTAGG |
| hsa-miR-21-5p_2sl: SEQ ID NO.34 | GAACAACCAACACAACCCAACATTGGTT GTTCTCAACA |
| hsa-miR-21-5p_2F: SEQ ID NO.35 | AGGGGTAGCTTATCAGACTGA |
| hsa-miR-21-5p_2R: SEQ ID NO.36 | AACAACCAACACAACCCAAC |
| hsa-miR-92a-3p_1sl: SEQ ID NO.37 | GGTTGTTGGTTGGTTGGTTGTATCCAAC AACCACAGGC |
| hsa-miR-92a-3p_1F: SEQ ID NO.38 | CCTCTATTGCACTTGTCCCG |
| hsa-miR-92a-3p_1R: SEQ ID NO.39 | GTTGTTGGTTGGTTGGTTGT |

### 3. In Vitro Functional Detection of Highly Abundant miRNAs in Exosomes

### (1) miRNA Mimics

miRNA mimics for cells and their control mimic NC were purchased from Guangzhou RiboBio Co., Ltd.
mimic NC (SEQ ID No.40): UUUGUACUACACAAAAGUACUG
miR-302a-3p (SEQ ID No.41): UAAGUGCUUCCAUGUUUUGGUGA
miR-302b-3p (SEQ ID No.42): UAAGUGCUUCCAUGUUUUAGUAG
miR-302c-3p (SEQ ID No.43): UAAGUGCUUCCAUGUUUCAGUGG
miR-302d-3p (SEQ ID No.44): UAAGUGCUUCCAUGUUUGAGUGU
miR-302a-5p (SEQ ID No.45): ACUUAAACGUGGAUGUACUUGCU
miR-21-5p (SEQ ID No.46): UAGCUUAUCAGACUGAUGUUGA
miR-92a-3p (SEQ ID No.47): UAUUGCACUUGUCCCGGCCUGU

### (2) Protective Effects of miRNA Mimics on Hepatocytes

To validate whether miRNAs in exosomes had protective effects on hepatocytes, an in vitro model of D-gal-induced injury in mouse primary hepatocytes (mPHs) was used. Some miRNAs from the top 10 miRNAs were selected as candidates. mPH were treated with 50 nM of the miRNA mimics. After 24 hours, cell viability in different groups was tested using a lactate dehydrogenase cytotoxicity assay kit. Results in FIG. 12 showed that miR-302 family members and miR-21-5p significantly inhibited D-gal-induced cell death. Among them, miR-302b-3p, miR-302d-3p, and miR-21-5p had particularly significant inhibitory effects, while miR-92a-3p had no protective effect. Since highly abundant miRNAs in exosomes were more likely key components responsible for their effects, we speculated that miR-302 family members (including miR-302a-3p, miR-302b-3p, miR-302c-3p, miR-302d-3p, and miR-302a-5p) and miR-21-5p, as highly abundant miRNAs in exosomes, might play major roles in treating acute liver failure in mice.

### V. Summary

In this example, a large number of exosomes derived from human embryonic stem cell-induced hepatocytes could be obtained by using the above-mentioned exosome extraction method. It was identified that the exosomes have complete morphology, and the particle size and distribution are within the range defined by exosomes. They have specific marker proteins to exosomes. Experiments have confirmed that the exosomes have a certain protective effect from hepatocyte damage in vivo and in vitro, can inhibit hepatocyte apoptosis and programmed necrosis, and reduce the inflammatory response in body, thereby playing a protective role in mice with liver failure, and providing a basis for its further functional research and application, and a new idea for the treatment of clinical acute liver failure or other liver-related diseases.

Technical features in the above embodiments may be combined in any combination. For simplicity of description, not all possible combinations of the technical features in the above embodiments are described. However, the combinations of the technical features are all to be considered as falling within the scope described in this specification provided that they do not conflict with each other.

The above examples only show several embodiments of the present disclosure, which are relatively specific and detailed, but should not be construed as limiting the scope of the present disclosure. It should be noted that various changes and modifications can be made by those of ordinary skill in the art without departing from the ideas of the present disclosure, and these changes and modifications are all within the scope of the present disclosure. Therefore, the scope of the present disclosure should be determined by the content of the appended claims.

## Claims

1. Use of exosomes in the manufacture of a medicament for treating a liver disease;
the exosomes are secreted by hepatocyte lineage cells differentiated via directed induction of embryonic stem cells, and the hepatocyte lineage cells are one or more of hepatic progenitor cells and mature hepatocytes.

2. Use according to claim 1, wherein the liver disease is acute liver failure.

3. Use according to claim 1 or 2, wherein the medicament comprises the exosomes and pharmaceutically acceptable excipients.

4. Use according to any one of claim 1 to 3, wherein the medicament is in the form of a tablet.

5. Use according to any one of claim 1 to 3, wherein the medicament is in the form of a capsule.

6. Use according to any one of claim 1 to 3, wherein the medicament is in the form of granules.

7. Use according to any one of claim 1 to 3, wherein the medicament is in the form of a suspension.

8. Use according to any one of claim 1 to 3, wherein the medicament is in the form of an oral solution.

9. Use according to any one of claim 1 to 3, wherein the medicament is in the form of an injection.

10. An in vitro method for protecting hepatocytes, comprising treating the hepatocytes to be protected with exosomes secreted by one or more of hepatic progenitor cells and mature hepatoid cells;
optionally, the hepatocytes to be protected are hepatocyte cell lines such as L02, HepG2, HuH-7, or mouse primary hepatocytes (mPHs),
optionally, the hepatocytes to be protected have cell damage induced by D-galactosamine,
optionally, the treatment is performed for about 24 hours to 48 hours,
optionally, the exosomes are used in a concentration of about 10 µg/mL to 100 µg/mL in the treatment.
